# EUROPEAN PATENT SPECIFICATION

(11) **EP 0 761 774 B1**
(45) Date of publication and mention of the grant of the patent: **07.05.2003**
(21) Application number: 95113988.0
(22) Date of filing: 06.09.1995
(51) Int. Cl.: C09C 1/04, C08K 9/06, A61K 7/42

(54) **Coated zinc oxide powder and coated zinc oxide powder-containing composition**
Beschichtetes Zinkoxidpulver und dieses enthaltende Zusammensetzung
Poudre d'oxide de zinc revêtu et composition la contenant

(43) Date of publication of application: 12.03.1997
(73) Proprietor: MITSUI MINING & SMELTING CO., LTD., Chuo-ku Tokyo-to 103 (JP)
(72) Inventor: Ichiryu, Akira, c/o Mitsui Mining and, Ageo-shi, Saitama (JP); Yamaguchi, Yasuhide, c/o Mitsui Mining and, Ageo-shi, Saitama (JP)
(74) Representative: Casalonga, Axel

(56) References cited:
- EP-A- 0 110 322
- EP-A- 0 255 304
- FR-A- 2 276 310
- US-A- 4 210 459
- US-A- 5 437 954
- DATABASE WPI Week 8302 Derwent Publications Ltd., London, GB; AN 83-03133K & JP-A-57 192 412 (CHISSO CORP.) , 26 November 1982
- PATENT ABSTRACTS OF JAPAN vol. 95 no. 1 & JP-A-07 003070 (MITSUBISHI GAS CHEM. CO.) 6 January 1995,
- DATABASE WPI Week 9118 Derwent Publications Ltd., London, GB; AN 91-130220 & JP-A-03 070 778 (KURARAY) , 26 March 1991
- DATABASE WPI Week 8707 Derwent Publications Ltd., London, GB; AN 87-047472 & JP-A-62 006 266 (RICOH) , 13 January 1987
- DATABASE WPI Week 8527 Derwent Publications Ltd., London, GB; AN 85-162563 & JP-A-60 093 455 (FUJI XEROX) , 25 May 1983
- DATABASE WPI Week 9534 Derwent Publications Ltd., London, GB; AN 95-261416 & JP-A-07 165 986 (TOKYO GAS CO.) , 27 June 1995
- PATENT ABSTRACTS OF JAPAN vol. 12 no. 297 (C-519) ,12 August 1988 & JP-A-63 063751 (SHISEIDO CO.) 22 March 1988,
- DATABASE WPI Week 8540 Derwent Publications Ltd., London, GB; AN 85-247118 & JP-A-60 163 950 (ASAHI CHEMICAL IND.) , 26 August 1985
- DATABASE WPI Week 7801 Derwent Publications Ltd., London, GB; AN 78-00600A & JP-A-52 135 739 (TOKYO SHIBAURA ELEC.) , 14 November 1977

## Description

### Technical Field to which the Invention Belongs

The present invention relates to zinc oxide powder used as an ultraviolet-absorbing pigment and more specifically to coated zinc oxide powder whose surface is treated and coated with a coupling agent, whose photocatalytic activity is inhibited, while maintaining various functions peculiar thereto such as ultraviolet-absorbing properties, antibacterial action, moldproof action and deodorizing action, which is improved in its dispersibility in resin compositions (such as paint and varnish, coated layers, films, molded resin articles and fibers) and oil and fat compositions (such as cosmetics) and which, as a result, permits effective prevention of deterioration, due to irradiation with light rays, in particular, ultraviolet rays, and storage stability of resin compositions and oil and fat compositions when it is incorporated into these compositions.

### Related Background Art

Resin compositions and oil and fat compositions which are organic substances undergo, for instance, decomposition, deterioration and discoloration due to irradiation with ultraviolet rays included in, for instance, solar rays. An ultraviolet-absorber is one of additives for preventing such decomposition, deterioration and discoloration of these compositions and is roughly divided into two groups, i.e., organic ultraviolet-absorbers represented by benzophenones and benzotriazoles; and inorganic ultraviolet-absorbers represented by zinc oxide powder and titanium oxide powder.

It has been well-known that the organic ultraviolet-absorber suffers from the following problems. The organic ultraviolet-absorber per se is deteriorated by the irradiation with ultraviolet rays since it is an organic substance and it also undergoes the so-called blooming in which the absorber oozes out to the surface of a coated film or a molded article with the lapse of time and is gradually washed away by, for instance, rainwater. For this reason, the inorganic ultraviolet-absorbers such as zinc oxide and titanium oxide have again attracted special interest recently. This is because they have an excellent ultraviolet-absorbing effect and can ensure protection of the earth environment (such tendency has been remarkable recently) since they are chemically stable and free of the blooming phenomenon. Moreover, it has been confirmed that they have not only an excellent ultraviolet-absorbing effect, but also other functions such as antibacterial, moldproof and deodorizing functions.

zinc oxide and titanium oxide exhibit an excellent ultraviolet-absorbing effect and therefore, permit the prevention of any deterioration of resin compositions and oil and fat compositions due to irradiation with ultraviolet rays when they are incorporated into these compositions, but they suffer from a problem such that they have a high photocatalytic activity induced by irradiation with ultraviolet rays and thus promote, for instance, decomposition, deterioration and discoloration of these compositions. More specifically, it has been well-known that the coated films and molded resin articles which comprise these inorganic ultraviolet-absorbers chalk, i.e., they undergo the so-called chalking phenomenon. Therefore, if zinc oxide and titanium oxide are used as ultraviolet-absorbers, the photocatalytic activity thereof should be inhibited by taking some measures.

As a means for solving this problem, titanium oxide is surface-coated with, for instance, Al₂O₃, SiO₂, ZnO and TiO₂ and such a coated titanium oxide product has already been put on the market. On the other hand, Japanese Un-Examined Patent Publication of the Translation of PCT Application (hereinafter referred to as "J.P. KOHYO") No. Hei 4-506674 discloses a technique for coating the surface of zinc oxide which comprises coating it with a water-insoluble metal soap of a monocarboxylic acid to give a zinc oxide product used as a pigment, which has ultraviolet-absorbing properties and is chemically inactive. However, the chemically inactivated zinc oxide used as a pigment disclosed in the J.P. KOHYO No. Hei 4-506674 is a pigment comprising surface-treated zinc oxide which is improved in stability to heat encountered during melting and kneading or press molding under heating while taking into consideration the description, in Examples 6 to 10, concerning the effect such that any deterioration of a resin was not observed during the kneading and molding. The surface-treated zinc oxide completely differs from the coated zinc oxide powder according to the present invention whose photocatalytic activity is inhibited and which thus serves to inhibit any deterioration of resin compositions and oil and fat compositions to which the coated zinc oxide is added. Moreover, Japanese Un-Examined Patent Publication (hereinafter referred to as "J.P. KOKAI") No. Hei 3-183620 discloses a technique for coating the surface of zinc oxide particles with an oxide or hydroxide of Al, Si, Zr or Sn, like the technique for coating the surface of titanium oxide.

The particle size of zinc oxide powder has increasingly been reduced and the specific surface area thereof accordingly becomes larger in response to a recent tendency of the market such that resin compositions and oil and fat compositions comprising an ultraviolet-absorber should have transparency and colorfulness. An increase in the specific surface area requires an increase in the amount of a coating material and accordingly, there has been a worry that an increased amount of a coating material is required for the inhibition of the photocatalytic activity of zinc oxide particles in the conventional surface-coating treatment with an oxide or hydroxide of Al, Si, zr or Sn and this would have a bad influence on the desired ultraviolet-absorbing effect thereof. More specifically, it has been urgent to develop a technique for surface-treatment which permits inhibition of the photocatalytic activity of zinc oxide while reducing the amount of a coating material, i.e., without reducing the relative amount of zinc oxide as much as possible.

Another problem, associated with the conventional techniques, to be solved is to improve the dispersibility of zinc oxide powder in resin compositions and oil and fat compositions. Ultraviolet-absorbers used as additives are dispersed in these compositions. The important property of the coated zinc oxide is an ultraviolet-absorbing effect observed when it is incorporated into these compositions and it has been well-known that the poorer the dispersibility of the coated zinc oxide particles and the stronger the agglomeration thereof, the faster the deterioration of the resin to which the coated zinc oxide is added. This is also true in case of coated titanium oxide particles. The conventional surface-coating treatment with an inorganic substance such as an oxide or hydroxide of Al, Si, Zr or Sn suffers from a problem concerning the dispersibility in resin compositions and oil and fat compositions. Accordingly, there has still been a worry that, even if zinc oxide is surface-treated through coating to inhibit the photocatalytic activity thereof, the desired ultraviolet-absorbing effect thereof is impaired when it is incorporated into resin compositions and oil and fat compositions because of its poor dispersibility and therefore, the coated zinc oxide never shows sufficient effect of preventing deterioration of the resin compositions and oil and fat compositions due to irradiation with light rays, in particular, ultraviolet rays.

### Disclosure of the Invention

Accordingly, an object of the present invention is the use of a coupling agent selected from the group consisting of silane type coupling agents, titanium type coupling agents, aluminium type coupling agents, zirconium type coupling agents and zircoaluminate type coupling agents, as a coating agent for ZnO particles to inhibit to a level of at most 2% the photocatalytic activity of those ZnO particles without negatively affecting one or more of its following properties :
- UV absorbing property
- antibacterial and anti-mold activity
- deodorizing activity, and
- good dispersability in resin, oil and fat compositions.
The coated zinc oxide powder serves as an ultraviolet-absorber, an antibacterial agent, a moldproof agent and a deodorizing agent when it is incorporated into resin compositions (such as paint and varnish, coated layers, films, molded resin articles and fibers) and oil and fat compositions (such as cosmetics) and it also serves to inhibit any photolytic deterioration of these compositions.

Another object of the present invention is therefore the use of ZnO particles coated with one or more of the coupling agents indicated above as an additive to a resin, oil or fat composition in order to protect that composition against UV light and/or bacterial and/or mold and/or odorizing activities.

The inventors of this invention have conducted various studies to accomplish the foregoing objects, have found out that the foregoing problems associated with the conventional techniques can effectively be solved by coating the surface of zinc oxide particles with a small amount of a coupling agent and thus have completed the present invention.

### Brief Description of the drawings

Fig. 1 is a graph showing the deodorizing effects of the coated zinc oxide powder prepared in Example 2 and uncoated zinc oxide powder of Comparative Example 4.
Fig. 2 is a graph showing the transmittance, as a function of wavelength (nm), of polyethylene films prepared in Example 5 and Comparative Examples 5, 7, 8 and 9.
Fig. 3 is a composition image (magnification: 40) of the film prepared in Example 5.
Fig. 4 is a composition image (magnification: 1000) of the film prepared in Example 5.
Fig. 5 is an EPMA (electron probe X-ray microanalyzer) image (magnification: 1000) of the film prepared in Example 5.
Fig. 6 is a composition image (magnification: 40) of the film prepared in Comparative Example 5.
Fig. 7 is a composition image (magnification: 1000) of the film prepared in Comparative Example 5.
Fig. 8 is an EPMA image (magnification: 1000) of the film prepared in Comparative Example 5.

### Description of Special Embodiment

Zinc oxide powder products used for preparing the coated zinc oxide powder according to the present invention may be those prepared by the so-called wet synthesis or those prepared by the so-called dry synthesis.

The surface of zinc oxide particles may be treated with a coupling agent by the so-called wet process in which a coupling agent is added to a slurry of zinc oxide powder in water or a non-aqueous solvent with stirring; or the so-called dry process wherein a coupling agent is sprayed on zinc oxide powder or dropwise added thereto with high speed stirring in a Henschel mixer or a high speed mixer capable of rotating at a high speed; or the so-called gas-phase process wherein the coating treatment is carried out by introducing a coupling agent along with an inert gas such as nitrogen gas as a carrier into a reaction vessel containing zinc oxide powder.

In this respect, however, the coated amount of the coupling agent should be determined while taking into consideration the specific surface area of zinc oxide powder to be coated. In Example 2 as will be detailed later, for instance, the photocatalytic activity of zinc oxide powder having a specific surface area, as determined by the BET method, of 20 m²/g could approximately completely be inhibited by coating with 1% by weight of a silane coupling agent. If finer zinc oxide powder having a specific surface area greater than that described above is subjected to the coating treatment for the same purpose, however, it would be easy to assume that the photocatalytic activity thereof cannot sufficiently be inhibited unless the coated amount of the coupling agent is increased while taking the foregoing rate of coating as a criterion.

More specifically, a coated amount on the order of about 20% is required for coating zinc oxide powder having extremely large specific surface area (for instance, 400 m²/g), while a coated amount on the order of about 0.1% would be sufficient for achieving a desired effect when treating, with a coupling agent, zinc oxide powder having a relatively large particle size, i.e., a small specific surface area on the order of several square meters per gram (m²/g). Therefore, the coated amount of the coupling agent in general ranges from 0.1 to 20% by weight, preferably 0.2 to 15% by weight while taking into consideration the dispersibility of the zinc oxide powder or preferably 0.1 to 10% by weight while taking into consideration the cost and preferably 0.2 to 10% by weight while considering the whole factors.

In Examples 1 to 3 as will be described later, there was used, as coating agent, silane coupling agent KBM-403 (γ - glycidoxy-propyltrimethoxy silane) or KBM-503 (γ - methacryloxypropyltrimethoxy silane) available from Shin-Etsu Chemical Co., Ltd. In this respect, it has been well-known that silanol groups formed by hydrolysis of the hydrolyzable groups present on the coupling agent mainly take part in the reaction with the surface of inorganic oxide particles such as zinc oxide particles, while organic functional groups such as epoxy groups and methacrylic groups mainly react with various kinds of resins to thus form linkages therebetween. The coupling agents which may be used for inhibiting the photocatalytic activity of the zinc oxide particles may be other silane coupling agents, i.e. those carrying, for instance, vinyl, mercapto and/or amino groups; or coupling agents other than silane coupling agents such as titanium, aluminum, zirconium and zircoaluminate type ones; or silanes.

The reason why the use of coupling agents as surface-coating agents permits the inhibition of the photocatalytic activity of zinc oxide in an amount smaller than that required when the conventional inorganic substances are used has not yet clearly been elucidated, but the coupling agent has reaction high selectivity to the active sites on the surface of zinc oxide particles, which contribute to the photocatalytic activity of the oxide particles, than that of the inorganic surface-treating agent to the active sites and therefore, a small amount of the coupling agent would serve to passivate the active sites which contribute to the photocatalytic activity, through the efficient use thereof without waste.

With regard to the coated zinc oxide powder according to the present invention, the photocatalytic activity thereof is efficiently inhibited using a small amount of a surface-treating agent, i.e., without severely reducing the relative content of zinc oxide. Therefore, the coated zinc oxide powder maintains the ultraviolet-absorbing function peculiar thereto and it also holds various other functions such as antibacterial,moldproof and deodorizing actions though the photocatalytic activity thereof is sufficiently inhibited. The reason why the coated zinc oxide powder shows such excellent effects has not yet been clearly elucidated, but it could be assumed that the active sites which contribute to the photocatalytic activity differ from those which contribute to antibacterial, moldproof and deodorizing actions of the zinc oxide particles. If such an assumption is true, the active sites which contribute to the foregoing functions would be well conserved and thus the coated zinc oxide maintains the functions if the zinc oxide particles are coated with a surface-treating agent in a smallest possible amount required for the passivation of the sites which contribute to the photocatalytic activity, i.e., in a smallest possible amount required for inhibiting the photocatalytic activity.

It is necessary to select an appropriate coupling agent while taking into consideration the compatibility of organic functional groups thereof to a resin used, in order to further improve the dispersibility of the coated zinc oxide powder in the resin. The improvement in the dispersibility of zinc oxide powder in resins cannot be accomplished by the conventional surface-coating treatment which makes use of an inorganic substance such as an oxide or a hydroxide of Al, Si, Zr or Sn. In Examples 4 to 6 as will be detailed below, for instance, zinc oxide powder whose surface was coated with KBM-503 was used since the resin component used therein was polyethylene.

The coated zinc oxide powder obtained through the use of the present invention can be used as an additive which has ultraviolet-absorbing properties as well as antibacterial, moldproof and deodorizing activity, but whose photocatalytic activity is almost completely inhibited. For instance, the coated powder is added to resin compositions or oil and fat compositions to impart ultraviolet-absorbing properties and antibacterial, mol dproof and deodorizing actions to the compositions. Moreover, the compositions are not decomposed, deteriorated and/or do not show discoloration since the photocatalytic activity of the additive is inhibited. In the present invention, if the coated zinc oxide powder is used as such an additive, it may be used alone or in the form of a mixture with other components.

If the additive of the invention is incorporated into paint and varnish, it may directly be added thereto or may be mixed with a component of the paint and varnish prior to the incorporation into the latter. The application of the paint and varnish thus prepared to walls, floors and ceilings permits the prevention of any discoloration and deterioration due to irradiation with ultraviolet rays included in the solar rays streaming in through windows and further permits deodorization and inhibition of mold-growth.

If the additive of the invention is kneaded with a resin composition, formed into a film and used as a packaging material for foods, the film prevents not only any discoloration of foods packed therein due to irradiation with ultraviolet rays, but also any decay thereof due to the antibacterial and moldproof activity and any emission of an offensive odor upon opening the package due to the deodorizing action thereof. Moreover, if the film-like material is used as, for instance, wallpaper, the coated zinc oxide incorporated therein permits the prevention of any discoloration and deterioration of the film-like material due to irradiation with ultraviolet rays included in the solar rays streaming in through windows and further permits deodorization and inhibition of mold-growth.

Furthermore, if the additive of the invention is kneaded with a resin composition and formed into a molded resin article or the additive is kneaded with a resins composition, followed by pulverizing into resin powder, then coating a base material with the powder to give a resin molded article such as kitchenwares, the additive permits the prevention of any decay of the articles and ensures the deodorization effect due to the antibacterial and moldproof actions thereof.

If the additive of the invention is incorporated into fibers by kneading it during the spinning process or by spinning fibers and then post-treating them through padding, the additive not only permits the prevention of any discoloration of fibers due to irradiation with ultraviolet rays, but also shows antibacterial, moldproof and deodorization effects.

If the additive is incorporated into cosmetics, it may be directly added to the cosmetics or may be loaded on the surface of, for instance, a powdery resin and then added to the cosmetics. The cosmetics thus prepared exhibit not only an ultraviolet-absorbing effect, but also deodorization effect, for instance,removal of the smell of sweat.

As has been discussed above in detail, the coated zinc oxide powder obtained by the use of the present invention is coated with a coupling agent in such a small amount that the coupling agent does not adversely affect the ultraviolet-absorbing, antibacterial, moldproof and deodorizing functions peculiar to the zinc oxide powder, that such coating treatment can sufficiently inhibit the photocatalytic activity of zinc oxide which may deteriorate resin compositions and oil and fat compositions when the powder is incorporated therein and that such coating treatment permits the improvement in the dispersibility of the powder in these compositions. Therefore, when the coated zinc oxide powder of the present invention is incorporated into a resin composition (such as coating layers, films, molded resin articles and fibers) or an oil and fat composition (such as cosmetics), it shows excellent functions as an ultraviolet-absorber, an antibacterial agent, a moldproof agent and a deodorizing agent and can inhibit any deterioration of resin compositions and oil and fat compositions.

The present invention will hereinafter be described in more detail with reference to the following non-limitative working Examples and Comparative Examples, but the present invention is not restricted to these specific Examples.

### Example 1

To an aqueous slurry of zinc oxide powder having a BET specific surface area of 20 m²/g, there was dropwise added, with stirring, a silane coupling agent: KBM-503 in such an amount that the coated amount thereof was equal to 0.5% by weight. The stirring was continued for a while, followed by filtration of the slurry, washing the recovered particles and drying with heating in a dryer to give dry powder of coated zinc oxide.

The coated zinc oxide powder prepared above was inspected for the photocatalytic activity in order to examine the photocatalytic activity-inhibitory effect of the surface-coating treatment with the silane coupling agent. The criterion, used herein, for evaluating the photocatalytic activity-inhibitory effect was a rate of decomposition of acetaldehyde through self-oxidation determined by introducing acetaldehyde gas and a predetermined amount of the foregoing coated zinc oxide powder into a transparent, sealed container, irradiating the container with ultraviolet rays and determining the rate of acetaldehyde-decomposition after a lapse of a predetermined period of time. The results thus obtained are listed in the following Table 1 together with the substance used for the surface-coating treatment and the coated amount thereof.

### Example 2

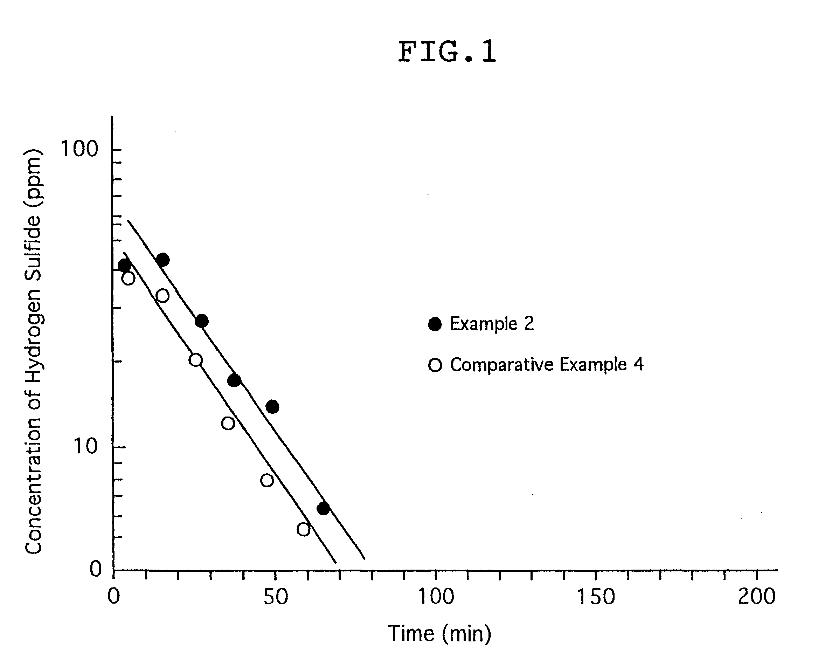

The same procedures as used in Example 1 were repeated except that a silane coupling agent: KBM-503 was used in such an amount that the coated amount thereof was equal to 1% to give dry powder of coated zinc oxide. Moreover, the photocatalytic activity-inhibitory effect thereof was evaluated by the same method as used in Example 1. The results thus obtained are listed in the following Table 1 together with the substance used for the surface-coating treatment and the coated amount thereof. Moreover, the coated zinc oxide powder prepared in this Example was inspected for the deodorization activity. The deodorization activity was evaluated by introducing hydrogen sulfide and the coated zinc oxide powder into a transparent, sealed container and determining the change, with time, in the concentration of the hydrogen sulfide remaining in the container. The results thus obtained are plotted on Fig. 1.

### Example 3

The same procedures as used in Example 1 were repeated except that a silane coupling agent: KBM-403 was used to give dry powder of coated zinc oxide. Moreover, the photocatalytic activity-inhibitory effect thereof was evaluated by the same method as used in Example 1. The results thus obtained are listed in the following Table 1 together with the substance used for the surface-coating treatment and the coated amount thereof.

### Comparative Examples 1 to 3

A desired amount of zinc oxide powder having a BET specific surface area of 20 m²/g was added to a sodium silicate aqueous solution in such a manner that the coated amount of SiO₂ was 3%, 7% or 18%, according to the method disclosed in J.P. KOKAI No. Hei 3-183620 and then a diluted hydrochloric acid solution was gradually added to the aqueous suspension while paying attention to the pH thereof. Then the addition of the diluted hydrochloric acid solution was interrupted at an instance when the pH of each suspension reached about 7 and it was allowed to stand for a while. Thereafter, each suspension thus obtained was filtered, followed by washing and drying with heating at 105 °C in a dryer to give each corresponding dry powder of coated zinc oxide. Moreover, the photocatalytic activity-inhibitory effect thereof was evaluated by the same method used in Example 1. The results thus obtained are listed in the following Table 1 together with the substances used for the surface-coating treatment and the coated amounts thereof.

### Comparative Example 4

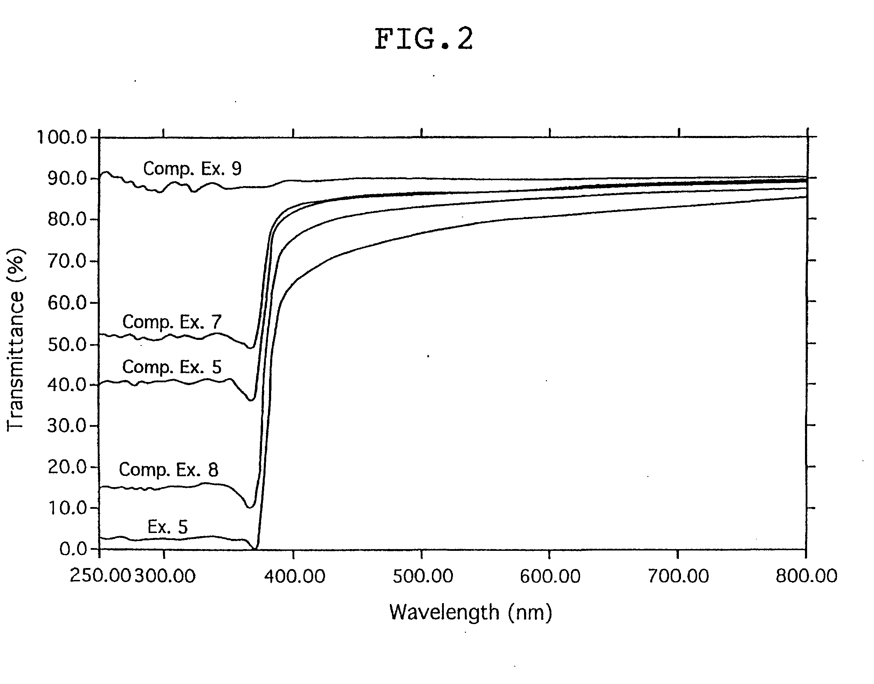

The uncoated zinc oxide powder having a BET specific surface area of 20 m²/g and used in Examples 1 to 3 and Comparative Examples 1 to 3, which had not been subjected to the surface-coating treatment, was used as a control for comparison. Moreover, the photocatalytic activity-inhibitory effect thereof was evaluated by the same method as used in Example 1. The result thus obtained is listed in the following Table 1. Furthermore, the deodorization effect thereof was also evaluated by the same method as used in Example 2. The results thus observed are plotted in Fig. 2.

**Table 1**

| Ex. No. | Substance Used for Surface-Coating | Coated Amount(%) | Rate of Decomposition of Acetaldehyde (%) |
|---|---|---|---|
| 1 | KBM-503 | 0.5 | 66.8 |
| 2 | KBM-503 | 1 | 0.9 |
| 3 | KBM-403 | 0.5 | 69.6 |
| 1 * | SiO₂ | 3 | 77.4 |
| 2 * | SiO₂ | 7 | 18.5 |
| 3 * | SiO₂ | 18 | 12.6 |
| 4 * | None | -- | 86.0 |

| | | | |
|---|---|---|---|
| *: Comparative Example | | | |

As seen from the data listed in Table 1, the uncoated zinc oxide powder prior to the surface-coating treatment shows a very high rate of acetaldehyde decomposition on the order of 86 %. This indicates that the uncoated zinc oxide powder has a very high photocatalytic activity. On the other hand, the coated zinc oxide powder of Example 2 (coated amount of KBM-503: 1%) according to the present invention shows a rate of acetaldehyde decomposition of 0.9% and this clearly indicates that the photocatalytic activity of zinc oxide which is a major obstacle when it is used as an ultraviolet-absorber can almost completely be inhibited.

In regard of the coated zinc oxide powder prepared in Comparative Examples 1 to 3, the rate of acetaldehyde-decomposition was found to be 77.4% at the coated amount of 3%, the powder shows almost no photocatalytic activity-inhibitory effect at this coated amount. The photocatalytic activity-inhibitory effect thereof is improved as the coated amount increases, but the rate of acetaldehyde-decomposition is in a matter of about 12.6% even at a coated amount of 18%. More specifically, the photocatalytic activity-inhibitory effect of the powder prepared in these Comparative Examples is substantially lower than that attained by the coated zinc oxide powder prepared in Example 2 in which the rate of decomposition is 0.9% at a coated amount of only 1%.

As has been discussed above, it has been confirmed that the zinc oxide powder coated with a coupling agent according to the present invention can almost completely inhibit the photocatalytic activity of zinc oxide powder at a substantially small coated amount as compared with the conventional techniques in which zinc oxide particles are coated with an inorganic substance.

Moreover, the graph shown in Fig. 1 which illustrates the deodorization effect indicates that the higher the slope of the straight line, the higher the deodorization effect of the coated zinc oxide powder and clearly shows that the coated zinc oxide powder prepared in Example 2 has a deodorization effect approximately identical to that of uncoated zinc oxide powder per se since the slope of the straight line showing the effect of the former is almost identical to that of the latter. In this respect, this test for evaluating deodorization effect was carried out without irradiation with ultraviolet rays, but approximately the same results were obtained even when the test was carried out under the irradiation with ultraviolet rays. The active sites which contribute to the photocatalytic activity would phenomenologically differ from those which contribute to antibacterial, moldproof and deodorizing actions while taking into consideration the fact that the zinc oxide powder exhibits the photocatalytic activity only when it is irradiated with ultraviolet rays. More specifically, in the coated zinc oxide powder, the coupling agent used for coating inhibits the photocatalytic activity of zinc oxide, but scarcely impairs the ultraviolet-absorbing effect as well as antibacterial, moldproof and deodorizing actions peculiar to the zinc oxide.

### Example 4

Three parts by weight of the coated zinc oxide powder prepared in Example 1 and 100 parts by weight of a polyethylene resin were melted and kneaded and then formed into a film having a thickness of 50 µm to thus inspect the film for the dispersibility of the coated zinc oxide powder in the polyethylene resin.

### Example 5

The same procedures as used in Example 4 were repeated except that the coated zinc oxide powder prepared in Example 2 was used to prepare a film and to evaluate the dispersibility of the powder in the resin. In addition, the resulting film was subjected to spectral transmission analysis to examine the ultraviolet-absorbing efficiency of the zinc oxide powder when it was incorporated into a resin composition. The results thus obtained are plotted on Fig. 2.

### Example 6

The same procedures as used in Example 4 were repeated except that the coated zinc oxide powder prepared in Example 3 was used to prepare a film and to evaluate the dispersibility of the powder in the resin.

### Comparative Example 5

The same procedures as used in Example 4 were repeated except that the coated zinc oxide powder prepared in Comparative Example 1 was used to prepare a film and to evaluate the dispersibility of the powder in the resin. In addition, the resulting film was subjected to spectral transmission analysis to examine the ultraviolet-absorbing efficiency of the zinc oxide powder when it was incorporated into a resin composition. The results thus obtained are plotted on Fig. 2.

### Comparative Example 6

The same procedures as used in Example 4 were repeated except that the coated zinc oxide powder prepared in Comparative Example 2 was used to prepare a film and to evaluate the dispersibility of the powder in the resin.

### Comparative Example 7

The same procedures as used in Example 4 were repeated except that the coated zinc oxide powder prepared in Comparative Example 3 was used to prepare a film and to evaluate the dispersibility of the powder in the resin. In addition, the resulting film was subjected to spectral transmission analysis to examine the ultraviolet-absorbing efficiency of the coated zinc oxide powder when it was incorporated into a resin composition. The results thus obtained are plotted on Fig. 2.

### Comparative Example 8

The same procedures as used in Example 4 were repeated except that the zinc oxide powder disclosed in Comparative Example 4 was used to form a film and to evaluate the dispersibility of the powder in the resin. In addition, the resulting film was subjected to spectral transmission analysis to estimate the ultraviolet-absorbing efficiency of the zinc oxide powder when it was incorporated into a resin composition. The results thus obtained are plotted on Fig. 2.

### Comparative Example 9

A film was formed by repeating the same procedures used in Example 4 except that the zinc oxide powder was not used. In addition, the resulting film was subjected to spectral transmission analysis to examine the ultraviolet-absorbing efficiency. The results thus obtained are plotted on Fig. 2.

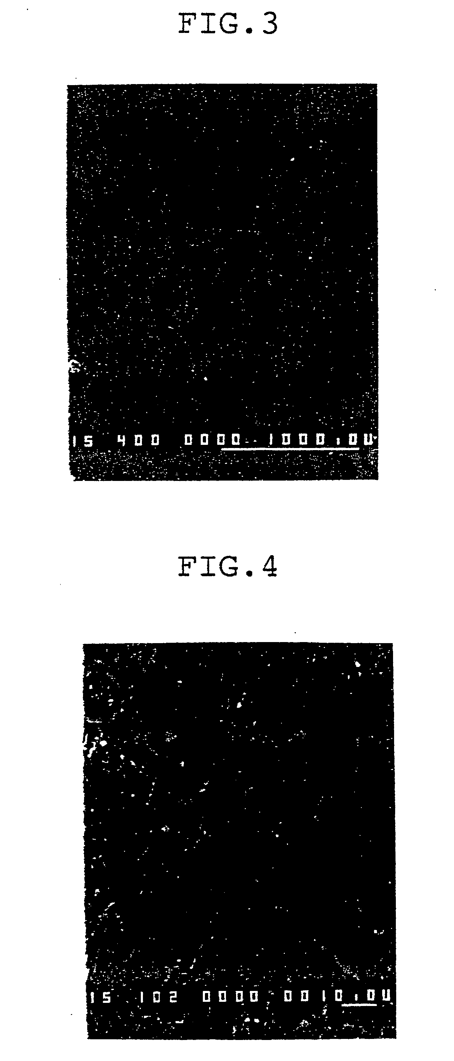

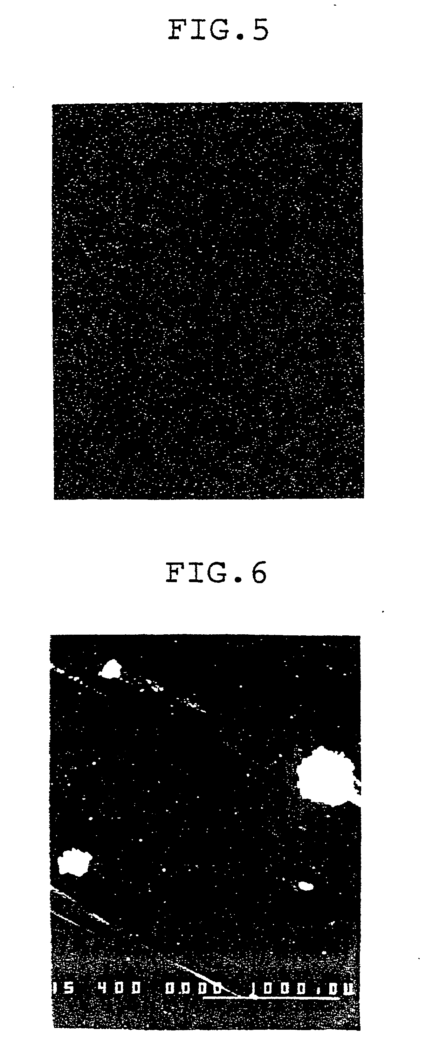

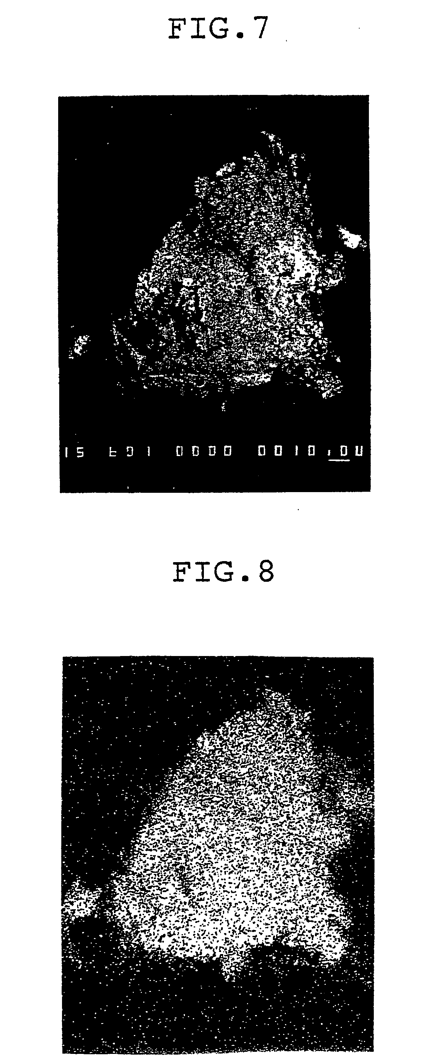

There was visually observed the presence of nondispersed agglomerates of zinc oxide particles in the films prepared in Comparative Examples 5 to 7 using zinc oxide powder coated with SiO₂ and the coated zinc oxide powder of these Comparative Examples had a poor dispersibility as compared with that observed for the film prepared using uncoated zinc oxide powder in Comparative Example 8. When comparing the films prepared in Examples 4 to 6 using the zinc oxide powder coated with the coupling agents with those prepared in Comparative Examples 5 to 8, the difference in the dispersibility between these films is clear from the visual observation thereof, but each film was further examined by taking a composition image (microgram showing the composition) and an EPMA image to confirm whether the agglomerates comprised zinc oxide or not. Fig. 3 is a composition image (magnification: 40) of the film prepared in Example 5; Fig. 4 is a composition image (magnification: 1000) of the film prepared in Example 5; Fig. 5 is an EPMA image (magnification: 1000) of the film prepared in Example 5; Fig. 6 is a composition image (magnification: 40) of the film prepared in Comparative Example 5; Fig. 7 is a composition image (magnification: 1000) of the film prepared in Comparative Example 5; and Fig. 8 is an EPMA image (magnification: 1000) of the film prepared in Comparative Example 5. As will be clear from Figs. 3 to 5, the coated zinc oxide powder of the present invention was uniformly dispersed throughout the film without forming any agglomerate. On the other hand, the coated zinc oxide powder of Comparative Example 5 formed large agglomerates in the film as seen from Figs. 6 to 8 and some of the agglomerates had a diameter of several hundred micrometers. This clearly indicates that the coated zinc oxide powder of Comparative Example 5 is inferior in the dispersibility in resins to the coated zinc oxide powder obtained according to the present invention.

In case of Comparative Example 8 (uncoated zinc oxide powder was used), there were observed so-called yellowing and discoloration during the kneading and molding steps. Contrary to this, the films prepared in Examples 4 to 6 did not undergo any yellowing and discoloration. This fact clearly indicates that the zinc oxide surface-treated using the method of the present invention is inhibited in not only the photocatalytic activity, but also activity thereof which would take part in the heat deterioration during kneading and molding steps. It was observed that the films prepared in Comparative Examples 5 to 7 underwent slight yellowing and discoloration. This also indicates that the present invention is superior to the conventional technique in the heat deterioration.

In Examples 4 to 6 and Comparative Examples 5 to 8, the dispersibility of zinc oxide powder was examined by kneading the powder with a resin, for convenience, but the same results can basically be obtained by preparing paint and varnish using the zinc oxide powder and then determining the dispersibility thereof. Moreover, the same results can likewise be obtained when the zinc oxide powder is dispersed in oils and fats.

As will be seen from the results shown in Fig. 2, the film of Example 5 according to the present invention can almost completely absorb ultraviolet rays (having a wavelength of not longer than 400 nm) and is not transparent to the ultraviolet rays. The film prepared in Example 5 comprises the zinc oxide powder coated with only 1% of a coupling agent, whose photocatalytic activity (a drawback of zinc oxide observed when it is used as an ultraviolet-absorber) is almost completely inhibited. The foregoing facts indicate that the coated zinc oxide powder obtained by the use of the present invention is satisfactorily inhibited in the photocatalytic activity, has improved dispersibility in resins and can function as an effective ultraviolet-absorber when incorporated into a resin composition, although the zinc oxide powder is coated with a coupling agent in a small amount which does not adversely affect the ultraviolet-absorbing efficiency.

On the other hand, the films of Comparative Examples 5 and 7 according to the conventional techniques have an ultraviolet-absorbing ability inferior to that observed for the film of Comparative Example 8 (uncoated zinc oxide powder is used). It would be assumed that this is because the dispersibility of the coated zinc oxide powder is reduced due to the surface-coating treatment with SiO₂ as has already been discussed above. More specifically, the conventional techniques suffer from a problem in that if the coated amount is increased to inhibit the photocatalytic activity, the dispersibility of the coated zinc oxide powder in a resin is greatly reduced when the powder is incorporated into a resin and this leads to a reduction of the ultraviolet-absorbing efficiency as is proved by the spectral curves of Comparative Examples 5 and 7 shown in Fig. 2. In short, the present invention has completely solved the problem associated with the conventional techniques through the use of a coupling agent.

### Example 7

To a slurry of zinc oxide powder (Comparative Example 4) in n-hexane, there was dropwise added, with stirring, a titanium coupling agent: KP TTS (available from Ajinomoto Co., Ltd.) in such an amount that the coated amount thereof was equal to 1% by weight. After continuing the stirring for a while, the slurry was filtered, followed by washing the recovered powder and drying in the air overnight to give dry powder of coated zinc oxide. The coated zinc oxide powder thus prepared was inspected for the photocatalytic activity-inhibitory effect by the same method as used in Example 1. Moreover, it was kneaded with a polyethylene resin, then formed into a film and the film was inspected for the dispersibility in resins by the same method used in Example 4. In addition, the ultraviolet-absorbing efficiency thereof was also determined in the same manner as used in Example 5. The results thus obtained are listed in the following Table 2 along with the substance used for the surface-coating and the coated amount thereof.

### Example 8

To a slurry of zinc oxide powder (Comparative Example 4) in water, there was dropwise added, with stirring, a zircoaluminate coupling agent M (available from CAVEDON CHEMICAL CO. INC.) in such an amount that the coated amount thereof was equal to 1% by weight. After continuing the stirring for a while, the slurry was filtered, followed by washing the recovered powder and drying with heating in a dryer to give dry powder of coated zinc oxide. The coated zinc oxide powder thus prepared was inspected for the photocatalytic activity-inhibitory effect by the same method as used in Example 1. Moreover, it was kneaded with a polyethylene resin, then formed into a film and the film was inspected for the dispersibility in resins by the same method as used in Example 4. In addition, the ultraviolet-absorbing efficiency thereof was also determined in the same manner as used in Example 5. The results thus obtained are listed in the following Table 2 along with the substance used for the surface-coating and the coated amount thereof.

The results obtained using the coated zinc oxide prepared in Example 2, those prepared in Comparative Examples 1 and 3 and the uncoated zinc oxide of Comparative Example 4 are also listed in the following Table 2.

**Table 2**

| Ex. No. | Substance Used for Surface-Coating Treatment | Coated Amount (%) | Photocatalytic Activity Inhibition¹⁾ (%) | Dispersibility in Resin²⁾ | Ultraviolet-Absorption Efficiency in Resin Comp.³⁾ (%) |
|---|---|---|---|---|---|
| 2 | silane coupling agent (KBM-503) | 1 | 0.9 | ⓞ | 2.7 |
| 7 | Ti-coupling agent (KR TTS) | 1 | 1.4 | ⓞ | 7.9 |
| 8 | zircoaluminate coupling agent | 1 | 2.0 | ⓞ | 3.7 |
| 1 * | SiO₂ | 3 | 77.4 | × | 40.7 |
| 3 * | SiO₂ | 18 | 12.6 | × | 51.0 |
| 4 * | None | -- | 86.0 | Δ | 14.9 |

| | | | | | |
|---|---|---|---|---|---|
| 1) The rate of acetaldehyde-decomposition. | | | | | |
| 2) The dispersibility in resin was evaluated on the basis of the number of agglomerates having a diameter of not less than about 30 µ m present within an area of 10 mm× 10 mm: ⓞ: no such agglomerate; ○ : less than 10; Δ : 10 to 20; × : more than 20 | | | | | |
| 3) The transmittance observed at 300 nm. | | | | | |
| *: Comparative Example | | | | | |

The data listed in Table 2 clearly indicate that the coated zinc oxide powder prepared in Examples 2, 7 and 8 according to the present invention are excellent ultraviolet-absorbers as compared with the coated zinc oxide powder prepared by the conventional techniques.

As has been described above, the present invention relates to the use of a coupling agent for coating zinc oxide powder and to the use of such coated zinc oxide powder. Incidentally, titanium oxide likewise suffers from a problem such that the photocatalytic activity thereof is stronger than that of zinc oxide powder when it is used as an ultraviolet-absorber and therefore, an effect similar to that achieved by the present invention would be expected if titanium oxide is subjected to a coating treatment with a coupling agent while taking into consideration the mechanism of the reaction between coupling agents and the surface of inorganic oxides.

## Claims

1. Use of a coupling agent selected from the group consisting of silane type coupling agents, titanium type coupling agents, aluminium type coupling agents, zirconium type coupling agents and zircoaluminate type coupling agents, as a coating agent for ZnO particles to inhibit to a level of at most 2% the photocatalytic activity of those ZnO particles without negatively affecting one or more of its following properties:
- UV absorbing property
- antibacterial and anti-mold activity
- deodorizing activity, and
- good dispersability in resin, oil and fat compositions.

2. Use of the coupling agent of claim 1 wherein this agent is a silane coupling agent.

3. Use of the coupling agent of claim 2 wherein the silane coupling agent is γ-glycidoxypropyltrimethoxy silane or γ-methacryloxypropyltrimethoxy silane.

4. Use of the coupling agent of claim 1, 2 or 3 wherein the coated amount of the coupling agent ranges from 0.1 to 20% by weight.

5. Use of the coupling agent of claim 4 wherein the coated amount of the coupling agent ranges from 0.2 to 10% by weight.

6. Use of ZnO particles coated with one or more of the coupling agents indicated in claims 1 to 5, as an additive to a resin, oil or far composition in order to protect that composition against UV light and/or bacterial and/or mold and/or odorizing activities.

## Patentansprüche

1. Verwendung eines Kupplungsmittels, ausgewählt aus der Gruppe, die aus Kupplungsmitteln vom Silantyp, Kupplungsmitteln vom Titantyp, Kupplungsmitteln vom Aluminiumtyp, Kupplungsmitteln vom Zirkoniumtyp und Kupplungsmitteln vom Zirkoaluminattyp besteht, als Beschichtungsmittel für ZnO-Teilchen, um die photokatalytische Aktivität von solchen ZnO-Teilchen bis zu einem Wert von höchstens 2 % zu hemmen, ohne negative Beeinflussung einer oder mehrerer seiner folgenden Eigenschaften:
- UV-Absorptionseigenschaft,
- antibakterielle Aktivität und Antipilzaktivität,
- Deodorierungsaktivität, und
- seine gute Dispergierbarkeit in Harz-, Öl- und Fettzusammensetzungen.

2. Verwendung des Kupplungsmittels nach Anspruch 1, bei welcher dieses Mittel ein Silan-Kupplungsmittel ist.

3. Verwendung des Kupplungsmittels nach Anspruch 2, bei welcher das Silan-Kupplungsmittel -Glycidoxypropyltrimethoxysilan oder -Methacryloxypropyltrimethoxysilan ist.

4. Verwendung des Kupplungsmittels nach Anspruch 1, 2 oder 3 bei welcher die aufgeschichtete Menge des Kupplungsmittels von 0,1 bis 20 Gew.% reicht.

5. Verwendung des Kupplungsmittels nach Anspruch 4, bei welcher die aufgeschichtete Menge des Kupplungsmittels von 0,22 bis 10 Gew.% reicht.

6. Verwendung von ZnO-Teilchen, beschichtet mit einem oder mehreren der in Anspruch 1 angegebenen Kupplungsmittel, als ein Zusatz zu einer Harz-, Öl- oder Fettzusammensetzung, um diese Zusammensetzung gegen UV-Licht und/oder bakterielle Aktivitäten und/oder Pilzaktivitäten und/oder Geruchsaktivitäten zu schützen.

## Revendications

1. Emploi d'un agent de couplage choisi parmi les agents de couplage de type silane, les agents de couplage au titane, les agents de couplage à l'aluminium, les agents de couplage au zirconium et les agents de couplage de type zircono-aluminate, comme agent de revêtement de particules d'oxyde de zinc, pour inhiber, jusqu'à un niveau de 2 % au maximum, l'activité photocatalytique de ces particules d'oxyde de zinc sans influencer négativement une ou plusieurs de leurs propriétés suivantes :
- propriété d'absorption du rayonnement ultraviolet,
- activité antibactérienne et anti-moisissures
- activité désodorisante,
- et bonne dispersabilité dans des compositions de résines, d'huiles ou de matières grasses.

2. Emploi d'un agent de couplage, conforme à la revendication 1, dans lequel cet agent est un agent de couplage de type silane.

3. Emploi d'un agent de couplage, conforme à la revendication 2, dans lequel l'agent de couplage de type silane est du γ-glycidyloxypropyltriméthoxy-silane ou du γ-méthacryloxypropyl-triméthoxy-silane.

4. Emploi d'un agent de couplage, conforme à la revendication 1, 2 ou 3, dans lequel la quantité d'agent de couplage utilisée dans le revêtement représente de 0,1 à 20 % en poids.

5. Emploi d'un agent de couplage, conforme à la revendication 4, dans lequel la quantité d'agent de couplage utilisée dans le revêtement représente de 0,2 à 10 % en poids.

6. Emploi de particules d'oxyde de zinc, revêtues de l'un ou plusieurs des agents de couplage indiqués dans les revendications 1 à 5, en tant qu'adjuvant dans une composition de résine, d'huile ou de matière grasse, afin de protéger cette composition contre les rayons ultraviolets et/ou l'activité de bactéries et/ou l'activité de moisissures et/ou le dégagement d'odeurs.
